# EUROPEAN PATENT APPLICATION

(11) **EP 2 669 374 A1**
(43) Date of publication of application: **04.12.2013**
(21) Application number: 12738922.9
(22) Date of filing: 26.01.2012
(51) Int. Cl.: C12N 15/09, C12Q 1/68, G01N 33/53, G01N 33/536, G01N 33/543, C07K 14/47

(54) **HUMAN IL-17-PRODUCING HELPER T CELL DETECTION MARKER AND HUMAN IL-17-PRODUCING HELPER T CELL DETECTION METHOD**

(30) Priority: 28.01.2011 JP 2011016753
(71) Applicant: Sysmex Corporation, Kobe-shi, Hyogo 651-0073 (JP)
(72) Inventor: MIYAMOTO, Yoshiaki, Kobe-shi Hyogo 651-0073 (JP); UGA, Hitoshi, Kobe-shi Hyogo 651-0073 (JP); KURATA, Hirokazu, Kobe-shi Hyogo 651-0073 (JP)
(74) Representative: Paemen, Liesbet R.J.
(86) International application number: PCT/JP2012/051638
(87) International publication number: WO 2012/102331

(57) **Abstract**

An object is to provide a marker that allows specific detection of human IL-17-producing helper T-cells (human Th17 cells).

The above problems are solved by a marker for detecting human Th17 cells containing a polynucleotide having a nucleotide sequence of the gene represented by at least one selected from the group consisting of L1CAM, MCAM and PTPRM or a variant or a fragment thereof or a protein encoded by the above gene or a functionally equivalent variant or a fragment thereof.

## Description

The present invention relates to a marker for detecting human IL-17-producing helper T-cells (hereinafter also referred to as "Th17 cells") and a method for detecting human Th17 cells.

### BACKGROUND ART

Rheumatoid arthritis (hereinafter referred to as "RA") is the systemic inflammatory autoimmune disease whose main clinical symptom is arthritis. The state of RA is diagnosed by subjective symptoms such as joint pain, the extent of swelling, and visual procedures such as the observations on bone X-ray. However, no quantitative index has been established. Thus, no quantitative method for continuously monitoring the therapeutic effects has been established under the current state of the art.

Details of the pathogenesis of RA have not been yet elucidated. It is considered that bacterial infections and the like trigger an inflammation in joint tissues via complicated networks of immunocyte group and cytokine group.
Helper T-cell group plays a central role in immune reactions. Immature helper T-cells (naive T-cells) are differentiated into helper T-cells when an antigen is presented by antigen-presenting cells. When specific cytokines are present at this time, naive T-cells are differentiated into four types of the cells. The four types of the cells are helper T-cells producing interferon (IFN)- γ (Th1 cells), helper T-cells producing interleukin (IL)-4 (Th2 cells), helper T-cells producing IL-17 (Th17 cells) and regulatory T-cells having immunosuppressive effects (Treg cells).

It has been shown that, among these helper T-cells, Th17 cells can be involved in the onset of RA. For example, Patent Document 1 shows that IL-17 is deeply involved in the formation of pathological conditions of RA, in particular, joint and bone deformities, because the level of IL-17 produced by Th17 cells is significantly higher in synovial fluid of RA patients than in that of the patients of osteoarthritis and T-cells in synovial tissue from RA patients include IL-17 positive cells. Further, Patent Document 1 describes that IL-17 can be used as a diagnostic marker of RA.

In addition, Patent Document 2 describes that the analysis of cytokines in peripheral blood serum of RA patients revealed that the levels of IFN- γ, IL-1β, TNF-α, G-CSF, GM-CSF, IL-6, IL-4, IL-10, IL-13, IL-5 and IL-7 were significantly high and the levels of IL-2, CXCL8/IL-8, IL-12 and CCL2/MCP-1 were not high in RA patients.

According to the studies by Non-Patent Document 1, Non-Patent Document 2 and Non-Patent Document 3, the following facts have been shown about Th17 cells:
- a nuclear receptor called RORγt has an important role in the differentiation of Th17 cells;
- IL-6, IL-23 and TGF-β induce the differentiation of immature helper T-cells (naive T-cells) to Th17 cells;
- they express IL-17A, IL-17F, IL-6, IL-22, IL-26, TNF, IFN- γ and CCL20; and
- IL-23 receptor and IL-12 receptor β are located on the surface of Th17 cells.

In Non-Patent Documents 1 to 3, the level of IL-17 is measured by enzyme linked immunosorbent assay (ELISA) using antibodies specific to IL-17. However, in order to more deeply understand the relations between RA and Th17 cells in autoimmune diseases, direct detection of the Th17 cells per se, not indirect measurement of the level of IL-17, is necessary.

### PRIOR ART DOCUMENTS

### PATENT DOCUMENTS

Patent Document 1: Japanese Patent Laid-open Publication No. 2000-186046
Patent Document 2: Japanese Patent Laid-open Publication No. 2007-506100

### NON-PATENT DOCUMENTS

Non-Patent Document 1: Ivanov et al., "The Orphan Nuclear Receptor RORγt Directs the Differentiation Program of Proinflammatory IL-17 + T Helper Cells", Cell, 2006, 126, p. 1121-1133
Non-Patent Document 2: Stumhofer et al., "Interleukin 27 negatively regulates the development of interleukin 17-producing T helper cells during chronic inflammation of the central nervous system" Nature Immunology, 2006, vol. 7, p. 937-945
Non-Patent Document 3: Wilson et al., "Development, cytokine profile and function of human interleukin 17-producing helper T cells" Nature Immunology, 2007, vol. 8, p. 950-957

### SUMMARY OF THE INVENTION

### PROBLEMS TO BE SOLVED BY THE INVENTION

An object of the present invention is to find a molecular marker that allows specific detection of human Th17 cells.

### SOLUTIONS TO THE PROBLEMS

The present inventors have focused on IL-17 producing helper T (Th17)-cells that are considered to be a cause of autoimmune diseases including RA, and so far identified a gene specifically expressed in Th17 cells isolated from peripheral blood of a healthy adult (International Patent Application PCT/JP2010/062807). This time, the present inventors have found combinations of markers that can distinguish Th17 cells and helper T-cells other than the Th17 cells from among these genes with high accuracy, thereby completing the present invention.

Thus, the present invention is a marker for detecting human Th17 cells which contains a polynucleotide having a nucleotide sequence of the gene represented by at least one selected from the group consisting of L1CAM (L1 cell adhesion molecule), MCAM (melanoma cell adhesion molecule) and PTPRM (protein tyrosine phosphatase, receptor type, M) or a variant or a fragment thereof.
Preferably, the marker further contains a polynucleotide having a nucleotide sequence of the gene represented by at least one selected from the group consisting of CCR6 (chemokine (C-C motif) receptor 6) and CXCR3 (chemokine (C-X-C motif) receptor 3) or a variant or a fragment thereof.
The present invention is also a marker for detecting human Th17 cells which contains a protein encoded by the gene represented by at least one selected from the group consisting of L1CAM, MCAM and PTPRM or a functionally equivalent variant or a fragment thereof.
Preferably, the marker further contains a protein encoded by the gene represented by at least one selected from the group consisting of CCR6 and CXCR3 or a variant or a fragment thereof.

Further, the present invention provides a method for detecting human Th17 cells comprising steps of obtaining the expression level of the marker for detecting human Th17 cells, in a sample containing cells, and detecting human Th17 cells in the sample, based on the expression level of the obtained marker.

### EFFECTS OF THE INVENTION

According to the marker for detecting human Th17 cells and the method for detecting human Th17 cells of the present invention, it is possible to specifically detect human Th17 cells.

### BRIEF DESCRIPTION OF THE DRAWINGS

Fig. 1 shows histograms showing the expression levels of the marker for detecting Th17 cells of the present invention (L1CAM, MCAM and PTPRM) and CCR6 that is a known marker, in Th1, Th2, Treg and Th17 cells.
Fig. 2 shows graphs that plot "the ratio of the number of cells expressed by the marker for detecting human Th17 cells of the present invention (%)" to "the ratio of Th17 cells detected by IL-17A (%)".
Fig. 3 shows a graph showing the concentration rates of Th17 cells when Th17 cells are isolated by the marker for detecting Th17 cells of the present invention from the cell population containing Th1, Th2, Treg and Th17 cells.

### EMBODIMENTS OF THE INVENTION

### [1. Marker for detecting human Th17 cells]

The marker for detecting human Th17 cells of the present invention (hereinafter, also simply referred to as "marker") contains a polynucleotide having a nucleotide sequence of the gene represented by at least one selected from the group consisting of L1CAM, MCAM and PTPRM or a protein encoded by the gene.

The present inventors have found that the marker of the present invention specifically expressed in Th17 cells rather than in other helper T-cells derived from human peripheral blood (Th1, Th2 and Treg cells).

As used herein, the phrase that a polynucleotide is "specifically expressed" in Th17 cells means that the expression of the polynucleotide in Th17 cells is significantly higher than the expression of the polynucleotide in cells other than Th17 cells.

Specifically, it means that the expression of the polynucleotide in Th17 cells is about two times or more and preferably about three times or more of the expression of the polynucleotide in cells other than Th17 cells. More preferably, the expression of the polynucleotide in Th17 cells is about two times or more and preferably about three times or more of the expression of the polynucleotide in helper T-cells other than Th17 cells (Th1 cells, Th2 cells and Treg cells).

Therefore, according to the marker of the present invention, it is possible to specifically detect Th17 cells distinctively from Th1, Th2 and Treg cells, and to study an index for activity of diseases in which Th17 cells may be involved.

The nucleotide sequences and amino acid sequences of the marker for detecting human Th17 cells can be known from the database provided by National Center for Biotechnology Information: NCBI such as UniGene. The accession numbers of the markers are shown in Table 1 below. These accession numbers are the latest number as of January 19, 2011.

**[Table 1]**

| Gene symbol | Entrez Gene ID | Protein ID | Transcript ID | UniGene ID |
|---|---|---|---|---|
| L1CAM | 3897 | NP_000416, NP_076493 | NM_000425, NM_024003 | Hs. 522818 |
| MCAM | 4162 | NP_006491 | NM_006500 | Hs. 599039 |
| PTPRM | 5797 | NP_001098714, NP_002836 | NM_001105244, NM_002845 | Hs. 49774 |

In an embodiment of the present invention, the marker for detecting human Th17 cells may be a polynucleotide marker or may be a polypeptide marker.
Therefore, the marker for detecting human Th17 cells is a polynucleotide having a nucleotide sequence of the gene represented by at least one selected from the group consisting of L1CAM, MCAM and PTPRM or a variant or a fragment thereof. Alternatively, the marker for detecting human Th17 cells is a protein encoded by the gene represented by at least one selected from the group consisting of L1CAM, MCAM and PTPRM or a functionally equivalent variant or a fragment thereof.
As used herein, the polynucleotide may be either DNA or RNA, and may be any of the gene per se (DNA), mRNA, cDNA or cRNA.

In an embodiment of the present invention, it is preferred that the marker for detecting human Th17 cells contain at least two selected from the group consisting of L1CAM, MCAM and PTPRM. In this case, the marker for detecting human Th17 cells may be a polynucleotide having a nucleotide sequence of the genes represented by at least two selected from the group consisting of L1CAM, MCAM and PTPRM or a variant or a fragment thereof, or a protein encoded by the gene represented by at least two selected from the group consisting of L1CAM, MCAM and PTPRM or a functionally equivalent variant or a fragment thereof.

In another embodiment of the present invention, the marker for detecting human Th17 cells may further contain a known marker used for detecting human Th17 cells in the art. It is known in the art that CCR6 is highly expressed ("CCR6 positive" or "CCR6+") and CXCR3 is not so expressed ("CXCR3 negative" or "CXCR3-") in human Th17 cells.
In the present embodiment, it is preferred that the marker for detecting human Th17 cells contain at least one selected from the group consisting of L1CAM, MCAM and PTPRM and at least one selected from the group consisting of CCR6 and CXCR3.

In the above embodiment, the marker for detecting human Th17 cells may be a polynucleotide having a nucleotide sequence of the gene represented by at least one selected from the group consisting of L1CAM, MCAM and PTPRM and at least one selected from the group consisting of CCR6 and CXCR3 or a variant or a fragment thereof. Alternatively, the marker for detecting human Th17 cells may be a protein encoded by the gene represented by at least one selected from the group consisting of L1CAM, MCAM and PTPRM and at least one selected from the group consisting of CCR6 and CXCR3 or a functionally equivalent variant or a fragment thereof.

As used herein, the variant of a polynucleotide means a polynucleotide into which a mutation that does not alter the function of the protein encoded by the above gene has been introduced. Such mutation includes a deletion or substitution of one or a plurality of nucleotides, or addition of one or a plurality of nucleotides in the nucleotide sequence of the above gene. The nucleotide sequence of the variant has at least 80% or more, preferably at least 85% or more, more preferably at least 90% or more and particularly preferably at least 95% or more sequence identity to the nucleotide sequence of the original gene.

As used herein, the functionally equivalent variant of a protein means a protein into which a mutation that does not alter the function of the protein has been introduced. Such mutation includes a deletion or substitution of one or a plurality of amino acids, or addition of one or a plurality of amino acids in the amino acid sequence of the above protein. The amino acid sequence of the functionally equivalent variant of a protein has at least 80% or more, preferably at least 85% or more, more preferably at least 90% or more and particularly preferably at least 95% or more sequence identity to the amino acid sequence of the original protein.
As used herein, the sequence identity of nucleotide sequence and amino acid sequence refers to those calculated using BLASTN, BLASTP, BLASTX or TBLASTN (e.g. available from http://www.ncbi.nlm.nih.gov) with default settings.

As used herein, the fragment of a polynucleotide means a polynucleotide having a continuous part of the nucleotide sequence of the marker for detecting human Th17 cells and having a length which allows its specific hybridization with a probe for obtaining the expression level of the marker for detecting human Th17 cells described later.
As used herein, the fragment of a protein means a polypeptide having a continuous part of the amino acid sequence of the marker for detecting human Th17 cells and having a length specifically recognized by an antibody or nucleic acid aptamer for obtaining the expression level of the marker for detecting human Th17 cells described later.

### [2. Method for detecting human Th17 cells]

A method for detecting human Th17 cells in a sample containing cells using the marker for detecting human Th17 cells is also within the scope of the present invention.

The method for detecting human Th17 cells of the present invention (hereinafter, also simply referred to as "detection method") includes steps of obtaining the expression level of the marker of the present invention in a sample containing cells and detecting human Th17 cells in the sample, based on the expression level of the obtained marker.

In an embodiment of the present invention, the sample containing cells includes a biological sample obtained from human or a sample containing an artificially cultured cell line. The biological sample includes blood, tissue, synovial fluid, cerebrospinal fluid, pleural fluid, ascitic fluid, and the like.

As used herein, "the expression level of the marker" is not particularly limited as long as it is the data showing the expression level of the markers obtained from a sample containing cells by the method known in the art.
When the marker for detecting human Th17 cells of the present invention is a polypeptide marker, the expression level of the marker includes the quantitative value of the protein that can be obtained by the method known in the art, for example, immunoprecipitation method, Western blotting, and ELISA, using an antibody or nucleic acid aptamer for obtaining the expression level of the marker described later.
When the marker for detecting human Th17 cells of the present invention is a polynucleotide marker, the expression level of the marker includes the quantitative value of the nucleic acid (DNA, mRNA, cDNA or cRNA) that can be obtained by the method known in the art, for example, nucleic acid amplification methods such as PCR, RT-PCR, real-time PCR, and LAMP (Loop-mediated isothermal amplification), hybridization methods such as Southern hybridization and Northern hybridization, and microarray. In addition, the expression level of the polynucleotide marker may be obtained using an antibody or nucleic acid aptamer on polynucleotide, as with the method for obtaining the expression level of the polypeptide marker described above.

In an embodiment of the present invention, a molecule that can specifically hybridize to the marker for detecting human Th17 cells as a polynucleotide marker can be used as a probe for obtaining the expression level of the marker (hereinafter, the probe may be also referred to as "probe for obtaining the expression level"). The probe for obtaining the expression level may be a nucleic acid probe and peptide probe that can specifically hybridize to the marker for detecting human Th17 cells as a polynucleotide marker. The probe is preferably a nucleic acid probe, and particularly preferably a DNA probe.

As used herein, the phrase "can specifically hybridize" means that the above probe can hybridize to the marker for detecting human Th17 cells as a polynucleotide marker under a stringent condition.
As used herein, stringent condition is a condition under which the above probe can hybridize to the target polynucleotide with a detectably higher extent than it does to a polynucleotide other than the target polynucleotide (e.g. more than at least two times of the background).
The stringent condition generally depends on the sequences and varies depending on various circumstances. Generally, the stringent condition is selected so that it is about 5°C lower than a thermal melting point: Tm of the specific sequence under a certain ionic strength and pH. This Tm is a temperature at which 50% of the complementary probe hybridizes to the nucleotide sequence of the target nucleic acid molecule in equilibrium (under a certain ionic strength, pH and nucleic acid composition).

Such condition may be a condition used in hybridization techniques between polynucleotides in hybridization techniques between polynucleotides known in the art, for example, PCR, microarray or Southern blotting. Specifically, it may be a condition of pH 7.0 to 9.0, a salt concentration of lower than about 1.5 M Na-ion, more specifically about 0.01 to 1.0 M Na-ion concentration (or other salt) and a temperature of at least about 30°C. For example, the stringent condition in microarray technique includes the hybridization at 37°C in 50% formamide, 1 M NaCl and 1% SDS and washing in 0.1 x SSC at 60 to 65°C. Also, the stringent condition in PCR technique includes a condition of pH 7.0 to 9.0, 0.01 to 0.1 M Tris-HCl, 0.05 to 0.15 M K-ion concentration (or other salt) and at least about 55°C.

The nucleotide sequence of the probe for obtaining the expression level can be appropriately determined by a person skilled in the art based on the common technical knowledge in the art and the nucleotide sequence of the marker for detecting human Th17 cells so that it can specifically hybridize to the marker. Such sequence can be determined by using, for example, a commonly available primer designing software (e.g. Primer3 (available from http://frodo.wi.mit.edu/cgi-bin/primer3/primer3.cgi) or DNASIS Pro (Hitachi Software Engineering Co., Ltd.)).
The probe for obtaining the expression level can be prepared according to polynucleotide synthesis methods known in the art. The length of the probe for obtaining the expression level is usually 5 to 50 nucleotides, and preferably 10 to 40 nucleotides.

As the probe for obtaining the expression level, only one probe is used, or a plurality of probes can be used in combination. For example, microarray for obtaining the expression level of the marker for detecting human Th17 cells can be prepared by immobilizing one or more probes on a substrate using a method known in the art.
The probe for obtaining the expression level can be combined with, for example, two or more primers for amplifying the marker for detecting human Th17 cells by a nucleic acid amplification method.

In an embodiment of the present invention, a molecule that can specifically bind to the marker for detecting human Th17 cells as a polypeptide marker can be used for obtaining the expression level of the marker. Such molecule may be either an antibody or an aptamer that specifically reacts with the marker for detecting human Th17 cells, and is preferably an antibody (hereinafter, the antibody is also referred to as "antibody for obtaining the expression level").

The antibody for obtaining the expression level can be prepared, for example, by the following procedures known in the art. A DNA molecule encoding a protein having an amino acid sequence of each marker is incorporated into an appropriate expression vector, based on the nucleotide sequence of the gene or the amino acid sequence of the protein of the marker for detecting human Th17 cells. The obtained expression vector is introduced into an appropriate host cells, and the obtained transformed cells are cultured to obtain a target protein. The obtained protein is purified and used as an immunogen optionally with an adjuvant to immunize an appropriate mammal such as rat or mouse. From spleen cells of the immunized animals and the like, antibody-producing cells that produce an antibody directed to the target immunogen are selected by screening. The obtained antibody-producing cells are fused with myeloma cells to obtain hybridomas. These hybridomas are screened, whereby antibody-producing hybridomas that produce an antibody having specific binding property to the protein encoded by the gene can be obtained. The antibody for obtaining the expression level can be obtained by culturing the obtained antibody-producing hybridomas.

The aptamer that can be used for obtaining the expression level of the marker for detecting human Th17 cells (hereinafter, the aptamer is also referred to as "aptamer for obtaining the expression level") can be prepared, for example, by the following procedures known in the art. A nucleic acid library including nucleotide sequences of random nucleic acid is prepared according to the known technique, and an aptamer that can specifically binds to the target protein (the marker for detecting human Th17 cells as protein or polypeptide) can be selected by the systematic evolution of ligands by exponential enrichment method (SELEX method) or the like.

The probe, antibody and aptamer for obtaining the expression level described above may be labeled with a labeling substance normally used in the art. Use of the labeled probe, antibody or aptamer allows an easy obtainment of the expression level of the marker for detecting human Th17 cells. Such labeling substance may be a labeling substance generally used in the art including radioisotopes such as ³²P, ³⁵S, ³H and ¹²⁵I, fluorescent substances such as fluorescein and Alexa Fluor (registered trademark), enzymes such as alkaline phosphatase and horseradish peroxidase, biotin, avidin, streptavidin, and the like.
When the expression level of the marker of the present invention is obtained using a flow cytometer, the labeling substance is preferably a fluorescent substance.

In an embodiment of the present invention, the expression level of the marker may be information relating to the cells that highly express or hardly express the polypeptide marker for detecting human Th17 cells of the present invention. Examples of such information include signals from a labeling substance of the labeled antibody bound to the marker of the present invention located on the surface of cells in a sample.
In a preferred embodiment of the present invention, the step of obtaining the expression level of the marker is steps of bringing a labeled antibody specifically reacting with a polypeptide marker for detecting human Th17 cells that is labeled with a labeling substance into contact with the sample containing cells to prepare a measurement sample, and detecting labeling of the labeled antibody bound to human Th17 cells through the polypeptide marker in the obtained measurement sample.
The labeled antibody can be obtained by directly binding a labeling substance to the antibody for obtaining the expression level, or using the above antibody as a primary antibody, binding a labeling secondary antibody specifically recognizing this primary antibody.

In the detection method of the present invention, it is preferred to obtain the expression levels of a plurality of the polypeptide markers for detecting human Th17 cells different from each other.
Therefore, in an embodiment of the present invention, it is preferred that the step of obtaining the expression level of the marker be a step of obtaining the expression levels of a plurality of the markers for detecting human Th17 cells different from each other. It is preferred that the above labeled antibody be an antibody different from each other that specifically reacts with each of a plurality of the markers for detecting human Th17 cells, and be labeled with mutually distinguishable labeling substances.
Here, the combination of mutually distinguishable labeling substances is known in the art, and examples include a combination of a red fluorescent substance with a green fluorescent substance and the like.

In an embodiment of the present invention, it is preferred that the labeled antibody be an antibody labeled with a fluorescent labeling substance.

The measurement sample in each embodiment described above can be prepared by bringing a labeled antibody into contact with a sample containing cells for an appropriate period of time. In the measurement sample, human Th17 cells, when exist, bind to the labeled antibody through the marker of the present invention on their surface. Moreover, labeling of the labeled antibody bound to the human Th17 cells in this measurement sample is detected, whereby information relating to cells that highly express or hardly express the polypeptide marker for detecting human Th17 cells of the present invention can be obtained.
When the labeled antibody is a fluorescently-labeled antibody, it is preferred that the detection of labeling from the measurement sample be performed by detecting fluorescence emitted from the fluorescently-labeled antibody using a flow cytometer. When using a flow cytometer, the human Th17 cells bound to the fluorescently-labeled antibody can be also isolated. Here, detection of fluorescence from a measurement sample using a flow cytometer is known in the art, and the conditions of detection and the like can be appropriately determined by a person skilled in the art.

### Examples

The present invention is described in detail by way of examples, and the present invention is not limited to these examples.

### (Example 1)

Analysis of highly expressed genes in cultured Th17 cells derived from human peripheral blood

### 1. Isolation of Th1, Th2, Treg and Th17 cells from human peripheral blood

### (1) Isolation of Th1, Th2 and Th17 cells from human peripheral blood

Buffy coat obtained from peripheral blood of a healthy adult was overlaid on Ficoll-paque plus solution (GE Healthcare Bio-Sciences KK) and centrifuged to obtain a monocyte fraction. Subsequently, CD4 positive cells were coarsely purified from the fraction by using magnetic beads bound to anti-CD4 antibody (Miltenyi Biotec K.K.).
The CD4 positive cells thus obtained were stained using the fluorescent labeling antibodies shown in Table 2 and then Th1, Th2 and Th17 cells were separated by a cell sorter (FACS Aria: Becton Dickinson and Company). Also, the separation was carried out with the gating as shown in Table 3.

**[Table 2]**

| Antigen | Fluorescence labeled substance | Clone | Manufacturer |
|---|---|---|---|
| CD4 | APC-Cy7 | RPA-T4 | BD biosciences |
| CD25 | PE-Cy7 | BC96 | eBioscience, Inc. |
| CXCR3 | Alexa Fluor (trademark) 488 | 1C6/CXCR3 | BD biosciences |
| CCR4 | APC | FAB1567A | R&D Systems, Inc. |
| CCR6 | PE | 11A9 | BD biosciences |

**[Table 3]**

| Cell | Gating |
|---|---|
| Th1 | CD4^{high} CD25^{low-negative} CXCR3⁺ CCR6⁻ CCR4⁻ |
| Th2 | CD4^{high} CD25^{low-negative} CXCR3⁻ CCR6⁻ CCR4⁺ |
| Th17 | CD4^{high} CD25^{low-negative} CXCR3⁻ CCR6⁺ CCR4⁺ |

For the detail of the sorting procedures, see the literature by Acosta-Rodriguez EV et al. (Surface phenotype and antigenic specificity of human interleukin 17-producing T helper memory cells., Nat Immunol., 2007, vol. 8, p. 639-646).

### (2) Isolation of Treg cells from human peripheral blood

CD4 positive cells obtained in the same manner as the above step (1) were stained with the fluorescent labeling antibodies shown in Table 4, then CD4high CD25high CD127internal-negative cells were purified as Treg cells by using the above cell sorter.

**[Table 4]**

| Antigen | Fluorescence labeled substance | Clone | Manufacturer |
|---|---|---|---|
| CD4 | FITC | OKT4 | eBioscience, Inc. |
| CD25 | PE-Cy7 | BC96 | eBioscience, Inc. |
| CD45RO | PE | UCHL1 | BioLegend, Inc. |
| CD127 | Alexa Fluor (trademark) 647 | HIL-7R-M21 | BD biosciences |

For the detail of the sorting procedures, see the literature by Weihong Liu et al. (CD127 expression inversely correlates with FoxP3 and suppressive function of human CD4+ T reg cells., J Exp Med. 2006, vol. 203, p. 1701-1711).

### 2. Cell culture

### (1) Th1, Th2 and Th17 cell cultures

Th1, Th2 and Th17 cells derived from adult peripheral blood obtained in the above step 1. (1) were respectively seeded in a 96-well plate at the density of 1.5 x 10⁵ cells/0.3 ml/well. As a medium, Yssel medium (IMDM, 1% human serum of AB-type, 0.25% BSA, 1.8 mg/l 2-aminomethanol, 40 mg/l transferrin, 5 mg/l insulin, 2 mg/l linoleic acid, 2 mg/l oleic acid, 2 mg/l palmitic acid, 1% penicillin/ streptomycin) was used.
In addition, for activation and proliferation of the above cells, magnetic beads coated with anti-CD2/3/28 antibody (Miltenyi Biotec K.K.) (hereinafter also referred to as "antibody beads") were added at 0.75 x 10⁵ per well. Then, cytokines and neutralizing antibodies suitable for differentiation culture of respective Th1, Th2 and Th17 cells were added, and the cells were cultured in an incubator at 37°C in an atmosphere of 5% CO₂. Cytokines and neutralizing antibodies used are shown in Table 5.

**[Table 5]**

| Cell | Cytokine | Neutralizing antibody (clone) |
|---|---|---|
| Th1 | IL-12, IL-2 | Anti-IL-4 antibody (MP4-25D2) |
| Th2 | IL-4, IL-2 | Anti-IFN-γ antibody (R4-6A2) |
| Th17 | TGF-β1, IL-6, IL-23, IL-21, IL-1β, TNF α, IL-2 | Anti-IL-4 antibody (MP4-25D2) |
| | | Anti-IFN-γ antibody (R4-6A2) |

The concentrations of the above cytokines were 50 ng/ml for IL-6 and 10 ng/ml for other than IL-6. Also, the concentrations of the above antibodies were 10 µg/ml for anti-IFN-γ antibody and 2.5 µg/ml for anti-IL-4 antibody.
The cytokines and neutralizing antibodies were obtained from R&D systems, Inc. and eBioscience, Inc., respectively.
After three days from the start of culture, the cells were diluted three-fold with the medium containing the above cytokines and antibodies and cultured for further seven days (10 days in total).

### (2) Treg cell culture

Treg cells obtained in the above step 1. (2) were cultured in the same manner in Yssel medium as the above step 2. (1) and activated using the antibody beads. Further, to the medium were added IL-2 and TGF-β1 (R&D systems Inc.) as cytokines, and anti-IFN-γ antibody, anti-IL-4 antibody (eBioscience, Inc.) and anti-IL-6 antibody (BD bioscience) as neutralizing antibodies.
These cytokines and neutralizing antibodies were used at the concentrations of 10 ng/ml and 5 µg/ml, respectively.
After three days from the start of culture, the cytokines and neutralizing antibodies were added at the same amounts as those at the start of the culture. After three days from the start of culture, the cells were diluted three-fold with the medium containing the above cytokines and antibodies and cultured for further seven days (10 days in total).

### 3. Extraction of total RNAs

In order to extract total RNAs from the cells obtained in the above step 2., RNeasy Plus Mini kit and RNeasy micro kit (QIAGEN N.V.) were used. The specific procedures were performed according to the attached instructions of the kits.

### 4. Expression analysis by microarray

Total RNAs (10 to 100 ng) extracted from the cells in the above step 3. were reverse-transcribed to cDNAs with Two-Cycle Target Labeling and Control Reagents (Affymetrix, Inc.), and further transcribed to biotinylated-cRNAs. Subsequently, the amplified biotinylated-cRNAs (20 µg) were fragmented. The specific procedures were performed according to the attached instruction of the kit.
The biotinylated-cRNAs derived from the cells as obtained above (15 µg) were added to GeneChip Human Genome U-133 Plus 2.0 Array (Affymetrix, Inc.) as samples, transferred to GeneChip Hybridization Oven 640 (Affymetrix, Inc.) and hybridized under the conditions of 45°C and 60 rpm for 16 hours.
After completion of the hybridization, the microarray was washed and fluorescence-labeled using GeneChip Fluidic Station 450 (Affymetrix, Inc.), and scanned using GeneChip Scanner 3000 7G (Affymetrix, Inc.) to obtain fluorescence intensity data.

### 5. Identification of genes specifically expressed in human Th17 cells

The data of fluorescence intensities obtained in the above step 4. were standardized using the expression analysis software GeneSpring Ver.10 (Agilent Technologies, Inc.) based on MAS5 algorithm. Then, relative fluorescence intensities of the genes from Th17 cells were compared with those from Th1, Th2 and Treg cells.
The number of samples used in the above gene selection step is shown in Table 6.

**[Table 6]**

| | Th1 | Th2 | Th17 | Treg |
|---|---|---|---|---|
| Without activation stimulation | 5 | 5 | 5 | 4 |
| With activation stimulation | 5 | 5 | 5 | 3 |

The genes whose relative fluorescence intensities in Th17 cells were three or more times higher than any of those of Th1, Th2 and Treg cells and which were significantly expressed (which showed "p value < 0.05" after ANOVA statistical analysis between four groups of relative fluorescence intensities in Th1, Th2, Treg and Th17 cells) were identified as the genes which were specifically expressed in Th17 cells.
As a result, 142 genes including L1CAM, MCAM and PTPRM were identified. The expression ratio of L1CAM, MCAM and PTPRM to each of Th1, Th2 and Treg cells in the Th17 cells is shown in Table 7.

**[Table 7]**

| Gene symbol | Activation stimulation | Expression ratio | | |
|---|---|---|---|---|
| | | Th17/Th1 | Th17/Th2 | Th17/Treg |
| L1CAM | Without stimulation | 8.5 | 9.4 | 5.1 |
| | With stimulation | 10.3 | 6.6 | 5.9 |
| MCAM | Without stimulation | 9.5 | 18.0 | 5.6 |
| | With stimulation | 12.8 | 24.4 | 4.3 |
| PTPRM | Without stimulation | 3.6 | 76.0 | 3.7 |
| | With stimulation | 4.1 | 66.0 | 4.1 |

### (Example 2)

Expression analysis of protein of the markers for detecting Th17 cells in cultured Th1, Th2, Treg and Th17 cells derived from human peripheral blood

### 1. Preparation of measurement samples

### (1) Preparation of MCAM measurement samples

To Th17 cells (5 x 10⁶ cells/ml) prepared in Example 1 under the paragraph "2. Cell culture" was added a phycoerythrin (PE)-labeled anti-MCAM antibody (Biolegend, Inc.) so as to have a final concentration of 1.25 µg/ml, and the mixture was reacted at 4°C for 20 minutes.
After the reaction, phosphate buffered saline (PBS) containing 0.5% BSA was added, and the mixture was centrifuged to collect Th17 cells, thereby washing the cells. The washed Th17 cells were suspended in PBS containing 0.5 µg/ml 7-amino-actinomycin D (7-AAD) and 0.5% BSA to prepare an MCAM measurement sample of Th17 cells (5 x 10⁶ cells/ml).
MCAM measurement samples of Th1 cells (5 x 10⁶ cells/ml), of Th2 cells (5 x 10⁶ cells/ml) and of Treg cells (5 x 10⁶ cells/ml) were prepared in the same manner as above except that Th1, Th2 and Treg cells were used instead of Th17 cells.
A negative control sample (5 x 10⁶ cells/ml) was prepared by adding a PE-labeled mouse IgG2a isotype control (Biolegend, Inc.) to a final concentration of 1.0 µg/ml instead of the PE-labeled MCAM antibody and reacting at 4°C for 20 minutes.

### (2) Preparation of PTPRM measurement samples

To Th17 cells (5 x 10⁶ cells/ml) prepared under the paragraph "2. Cell culture" described above was added an anti-PTPRM antibody (Abcam plc) so as to have a final concentration of 2.0 µg/ml, and the mixture was reacted at 4°C for 20 minutes.
After the reaction, PBS containing 0.5% BSA was added, and the mixture was centrifuged to collect Th17 cells. The collected Th17 cells were suspended in PBS containing 0.5% BSA. This suspension was added with a PE-labeled anti-mouse IgG antibody (Biolegend, Inc.) so as to have a final concentration of 1.0 µg/ml, and the mixture was reacted at 4°C for 20 minutes.
After reaction with the PE-labeled anti-mouse IgG antibody, PBS containing 0.5% BSA was added, and the mixture was centrifuged to collect Th17 cells, thereby washing the cells. The washed Th17 cells were suspended in PBS containing 0.5 µg/ml 7-ammo-actinomycin D (7-AAD) and 0.5% BSA to prepare a PTPRM measurement sample of Th17 cells (5 x 10⁶ cells/ml).
PTPRM measurement samples of Th1 cells (5 x 10⁶ cells/ml), of Th2 cells (5 x 10⁶ cells/ml) and of Treg cells (5 x 10⁶ cells/ml) were prepared in the same manner as above except that Th1, Th2 and Treg cells were used instead of Th17 cells.
A negative control sample (5 x 10⁶ cells/ml) was prepared by adding a mouse IgG2a isotype control (Biolegend, Inc.) so as to have a final concentration of 1.0 µg/ml instead of the anti-PTPRM antibody, and reacting at 4°C for 20 minutes.

### (3) Preparation of L1CAM measurement samples

To Th17 cells (5 x 10⁶ cells/ml) prepared under the paragraph "2. Cell culture" described above was added an anti-L1CAM antibody (BD biosciences) so as to have a final concentration of 1.25 µg/ml, and the mixture was reacted at 4°C for 20 minutes.
After the reaction, PBS containing 0.5% BSA was added, and the mixture was centrifuged to collect Th17 cells. The collected Th17 cells were suspended in PBS containing 0.5% BSA. This suspension was added with an APC-labeled anti-mouse IgG antibody (BD biosciences) so as to have a final concentration of 1.0 µg/ml, and the mixture was reacted at 4°C for 20 minutes.
After reaction with the APC-labeled anti-mouse IgG antibody, PBS containing 0.5% BSA was added, and the mixture was centrifuged to collect Th17 cells, thereby washing the cells. The washed Th17 cells were suspended in PBS containing 0.5 µg/ml 7-amino-actinomycin D (7-AAD) and 0.5% BSA to prepare an L1CAM measurement sample of Th17 cells (5 x 10⁶ cells/ml).
L1CAM measurement samples of Th1 cells (5 x 10⁶ cells/ml) and of Th2 cells (5 x 10⁶ cells/ml) were prepared in the same manner as above except that Th1 cells and Th2 cells were used instead of Th17 cells.
A negative control sample (5 x 10⁶ cells/ml) was prepared by adding a mouse IgG2a isotype control (Biolegend, Inc.) so as to have a final concentration of 1.0 µg/ml instead of the anti-L1CAM antibody and reacting at 4°C for 20 minutes.

### (4) Preparation of CCR6 measurement samples

CCR6 measurement samples of Th17 cells (5 x 10⁶ cells/ml), of Th1 cells (5 x 10⁶ cells/ml), of Th2 cells (5 x 10⁶ cells/ml) and of Treg cells (5 x 10⁶ cells/ml) were prepared in the same manner as the above paragraph "(1) Preparation of MCAM measurement samples" except that a PE-labeled anti-CCR6 antibody (BD biosciences) at a final concentration of 1.0 µg/ml was used instead of the PE-labeled anti-MCAM antibody.
A negative control sample (5 x 10⁶ cells/ml) was prepared by adding a PE-labeled mouse IgG1 isotype control (Biolegend, Inc.) so as to have a final concentration of 1.0 µg/ml instead of the PE-labeled anti-CCR6 antibody and reacting at 4°C for 20 minutes.

### 2. Expression analysis of polypeptide markers in measurement samples using flow cytometer

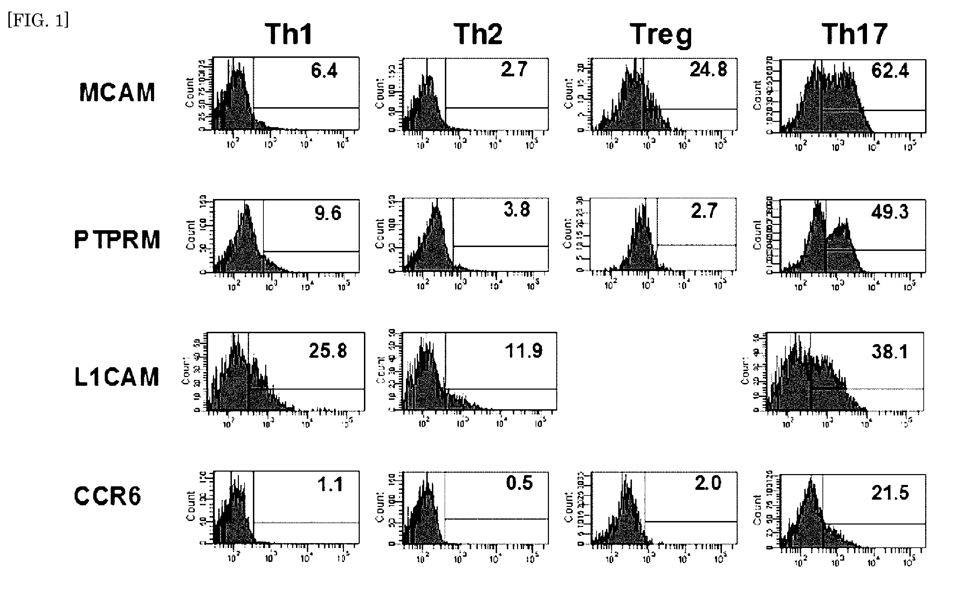

The MCAM measurement samples, PTPRM measurement samples, L1CAM measurement samples, and CCR6 measurement samples prepared as described above were analyzed using FACSCanto II (BD biosciences) and FACS DIVA software (BD biosciences). Histograms (particle size distribution) of fluorescence intensities obtained by the analysis are shown in Fig. 1. In Fig. 1, the vertical axis of the histograms shows the number of cells, and the horizontal axis shows the fluorescence intensity. Also, the numbers at the upper right of the histograms show the ratio (%) of the number of positive cells for the marker gene relative to the number of total cells in the respective measurement samples. The cells were determined as a positive cell or negative cell based on the maximum fluorescence intensity in the negative control. Namely, the cells showing higher fluorescence intensity than the maximum fluorescence intensity in the negative control were determined as a positive cell, while the cells showing a fluorescence intensity equal to or lower than the maximum fluorescence intensity in the negative control were determined as a negative cell. The ratio of positive cells was calculated as the ratio of the number of positive cells relative to the number of total cells.
Fig. 1 shows that MCAM, PTRRM and L1CAM are specifically highly expressed in Th17 cells as compared to in Th1 cells, Th2 cells and Treg cells. It is also found that the ratios of positive cells in the MCAM measurement sample, PTPRM measurement sample and L1CAM measurement sample of Th17 cells were higher than the case of positive cells in the CCR6 measurement sample that is a known marker. This reveals that the proteins encoded by the genes represented by MCAM, PTPRM and L1CAM are available as the polypeptide markers for detecting Th17 cells.

### (Example 3)

Correlation analysis with expression of protein of the markers for detecting Th17 cells in cultured Th1, Th2, Treg and Th17 cells derived from human peripheral blood

### 1. Sample preparation for measurement by polypeptide marker set

### (1) Preparation of mixed samples of Th cells

Th1 cells, Th2 cells, Treg cells and Th17 cells prepared under the paragraph "2. Cell culture" described above were each mixed in the ratio shown in the following Table 8 to prepare mixed samples of Th cells (10 types). Preparation of the mixed samples of Th cells were independently carried out to prepare a total of 40 samples (since data for 2 samples among 40 samples could not obtained, the analysis described later was finally performed using data of 38 samples).

**[Table 8]**

| Th cells mixed sample | Mixing rate of each Th cell | | | |
|---|---|---|---|---|
| | Th1 | Th2 | Treg | Th17 |
| 1 | 0.5 | 1 | 1 | 1 |
| 2 | 0 | 1 | 1 | 1 |
| 3 | 0.5 | 0 | 1 | 1 |
| 4 | 0.5 | 1 | 0 | 1 |
| 5 | 0.5 | 0 | 0 | 1 |
| 6 | 0.5 | 1 | 0 | 0 |
| 7 | 0 | 1 | 0 | 1 |
| 8 | 0.5 | 0 | 0 | 0 |
| 9 | 0 | 1 | 0 | 0 |
| 10 | 0 | 0 | 0 | 1 |

### (2) Preparation of PTPRM/MCAM polypeptide marker measurement sample

To the mixed sample of Th cells (5 x 10⁶ cells/ml) prepared under the paragraph "1-(1). Preparation of measurement samples" described above was added a final concentration of 2.0 µg/ml of an anti-PTPRM antibody (Abcam plc), and the mixture was reacted at 4°C for 20 minutes.
After the reaction, PBS containing 0.5% BSA was added, and the mixture was centrifuged to collect Th cells. The collected Th cells were suspended in PBS containing 0.5% BSA. This suspension was added with an APC-labeled anti-mouse IgG antibody (Biolegend, Inc.) so as to have a final concentration of 1.0 µg/ml, and the mixture was reacted at 4°C for 20 minutes.
After reaction with the APC-labeled anti-mouse IgG antibody, PBS containing 0.5% BSA was added, and the mixture was centrifuged to collect Th cells, thereby washing the cells.
To the washed Th cells was added a PE-labeled anti-MCAM antibody (Biolegend, Inc.) so as to have a final concentration of 1.25 µg/ml, and the mixture was reacted at 4°C for 20 minutes.
After the reaction, PBS containing 0.5% BSA was added, and the mixture was centrifuged to collect Th cells, thereby washing the cells.
The washed Th cells were suspended in PBS containing 0.5 µg/ml 7-amino-actinomycin D (7-AAD) and 0.5% BSA to prepare a PTPRM/MCAM polypeptide marker measurement sample (5 x 10⁶ cells/ml).

### (3) Preparation of PTPRM/L1CAM polypeptide marker measurement sample

A PE labeled anti-L1CAM antibody (eBioscience, Inc.) was added, instead of an anti-MCAM antibody under the paragraph "(2) Preparation of PTPRM/MCAM measurement samples" described above, so as to have a final concentration of 1.25 µg/ml, and the mixture was reacted at 4°C for 20 minutes.
After the reaction, PBS containing 0.5% BSA was added, and the mixture was centrifuged to collect Th cells, thereby washing the cells. The washed Th cells were suspended in PBS containing 0.5 µg/ml 7-amino-actinomycin D (7-AAD) and 0.5% BSA to prepare a PTPRM/L1CAM polypeptide marker measurement sample (5 x 10⁶ cells/ml).

### (4) Preparation of PTPRM/CCR6 polypeptide marker measurement sample

A PE labeled anti-CCR6 antibody (BD biosciences) was added, instead of an anti-MCAM antibody under the paragraph "(2) Preparation of PTPRM/MCAM measurement samples" described above, so as to have a final concentration of 1.0 µg/ml, and the mixture was reacted at 4°C for 20 minutes.
After the reaction, PBS containing 0.5% BSA was added, and the mixture was centrifuged to collect Th cells, thereby washing the cells. The washed Th cells were suspended in PBS containing 0.5 µg/ml 7-amino-actinomycin D (7-AAD) and 0.5% BSA to prepare a PTPRM/CCR6 polypeptide marker measurement sample (5 x 10⁶ cells/ml).

### (5) Preparation of PTPRM/CXCR3 polypeptide marker measurement sample

An Alexa488 labeled anti-CXCR3 antibody (BD biosciences) was added, instead of an anti-MCAM antibody under the paragraph "(2) Preparation of PTPRM/MCAM measurement samples" described above, so as to have a final concentration of 1.0 µg/ml, and the mixture was reacted at 4°C for 20 minutes.
After the reaction, PBS containing 0.5% BSA was added, and the mixture was centrifuged to collect Th cells, thereby washing the cells. The washed Th cells were suspended in PBS containing 0.5 µg/ml 7-amino-actinomycin D (7-AAD) and 0.5% BSA to prepare a PTPRM/CXCR3 polypeptide marker measurement sample (5 x 10⁶ cells/ml).

### (6) Preparation of CCR6/CXCR3 polypeptide marker measurement sample

A PE labeled anti-CCR6 antibody (BD biosciences) was added, instead of an anti-PTPRM antibody under the paragraph "(5) Preparation of PTPRM/CXCR3 measurement samples" described above, so as to have a final concentration of 1.0 µg/ml, and the mixture was reacted at 4°C for 20 minutes. In this case, the treatment using a secondary antibody (APC-labeled anti-mouse IgG antibody) under the paragraph "(5) Preparation of PTPRM/CXCR3 measurement samples" was not performed.
After the reaction, PBS containing 0.5% BSA was added, and the mixture was centrifuged to collect Th cells, thereby washing the cells. The washed Th cells were suspended in PBS containing 0.5 µg/ml 7-amino-actinomycin D (7-AAD) and 0.5% BSA to prepare a CCR6/CXCR3 polypeptide marker measurement sample (5 x 10⁶ cells/ml).

### (7) Preparation of CCR6/MCAM polypeptide marker measurement sample

An Alexa488 labeled anti-CCR6 antibody (Biolegend, Inc.) was added, instead of an anti-PTPRM antibody under the paragraph "(2) Preparation of PTPRM/MCAM measurement samples" described above, so as to have a final concentration of 1.0 µg/ml, and the mixture was reacted at 4°C for 20 minutes. In this case, the treatment using a secondary antibody (APC-labeled anti-mouse IgG antibody) under the paragraph "(2) Preparation of PTPRM/MCAM measurement samples" was not performed.
After the reaction, PBS containing 0.5% BSA was added, and the mixture was centrifuged to collect Th cells, thereby washing the cells. The washed Th cells were suspended in PBS containing 0.5 µg/ml 7-amino-actinomycin D (7-AAD) and 0.5% BSA to prepare a CCR6/MCAM polypeptide marker measurement sample (5 x 10⁶ cells/ml).

### (8) Preparation of CCR6/L1CAM polypeptide marker measurement sample

A PE labeled anti-L1CAM antibody (eBioscience) was added, instead of an anti-MCAM antibody under the paragraph "(7) Preparation of CCR6/MCAM measurement samples" described above, so as to have a final concentration of 1.0 µg/ml, and the mixture was reacted at 4°C for 20 minutes.
After the reaction, PBS containing 0.5% BSA was added, and the mixture was centrifuged to collect Th cells, thereby washing the cells. The washed Th cells were suspended in PBS containing 0.5 µg/ml 7-amino-actinomycin D (7-AAD) and 0.5% BSA to prepare a CCR6/L1CAM polypeptide marker measurement sample (5 x 10⁶ cells/ml).

### 2. Sample preparation for measurement by known marker of each Th cell

### (1) Preparation of IFN- γ, IL-4 and IL-17A measurement samples

The mixed samples of Th cells prepared under the paragraph "1. (1) Preparation of mixed samples of Th cells" were prepared to be 2.5 x 10⁵ cells/ml with 5% FBS/RPMI. Subsequently, phorbol myristate acetate and ionomycin were added so as to have a final concentration of 50 ng/ml and a final concentration of 1 µM, respectively, and incubated at 37°C for 4 hours to stimulate Th cells. Then, brefeldin A was added so as to have a final concentration of 10 µg/ml and incubated at 37°C for 2 hours.
After cultivation, phosphate buffered saline (PBS) containing 0.5% BSA was added, and the mixture was centrifuged to collect Th cells, thereby washing the cells. The washed Th cells were added with 2% paraformaldehyde to fix the cells. After fixing the cells, a saponin buffer (0.5% saponin, 0.5% bovine serum albumin (BSA), 1 mM sodium azide (in PBS)) was added to accelerate cell membrane permeability of Th cells.
To the sample after saponin buffer treatment were added a PerCP-Cy5.5-labeled anti-IL-17A antibody (eBioscience, Inc.) at a final concentration of 0.15 µg/ml, an APC-labeled anti-IL-4 antibody (eBioscience, Inc.) at a final concentration of 0.2 µg/ml and an Alexa488 labeled anti-IFN-γ antibody (Biolegend, Inc.) at a final concentration of 1.0 µg/ml, and the mixture was reacted at 4°C for 20 minutes. Here, IFN-γ was used as a known marker for detecting Th1 cells, IL-4 was used as a known marker for detecting Th2 cells and IL-17A was used as a known marker for detecting Th17 cells, respectively.
After the reaction, the saponin buffer was added, and the mixture was centrifuged to collect Th cells, thereby washing the cells. The washed Th cells were suspended in PBS containing 0.5% BSA to prepare IFN-γ, IL-4 and IL-17A measurement samples (2.5 x 10⁵ cells/ml).

### (2) Preparation of FOXP3 measurement sample

The mixed sample of Th cells prepared under the paragraph "1. (1) Preparation of mixed samples of Th cells" described above were fixed and subjected to membrane permeation treatment using FOXP3 staining buffer set (eBioscience, Inc.), then a PE-labeled anti-FOXP3 antibody (Biolegend, Inc.) was added so as to have a final concentration of 3.125 µg/ml, and the mixture was reacted at 4°C for 20 minutes. Here, FOXP3 was used as a known marker for detecting Treg cells.
After reaction, phosphate buffered saline (PBS) containing 0.5% BSA was added, and the mixture was centrifuged to collect Th cells, thereby washing the cells. The washed Th cells were suspended in PBS containing 0.5% BSA to prepare a FOXP3 measurement sample (5 x 10⁶ cells/ml).

### 3. Expression analysis of polypeptide marker set in measurement samples and Th cell known marker using flow cytometer

The respective polypeptide marker measurement samples prepared in 1. (2) to (8) described above and IFN-γ, IL-4, IL-17A and FOXP prepared in 2. (1) to (2) described above were analyzed using FACSCanto II (BD biosciences) and FACS DIVA software (BD biosciences). The ratio (%) of the number of cells expressing polypeptide marker shown in Table 9 below to the number of total cells in the respective polypeptide marker measurement samples was calculated. In addition, in IFN-γ, IL-4, IL-17A and FOXP3 measurement samples, the ratios of the number of IFN-γ positive cells, the number of IL-4 positive cells, the number of IL-17A positive cells and the number of FOXP3 positive cells to the number of total cells were defined as the ratio of Th1 cells (%), the ratio of Th2 cells (%), the ratio of Th17 cells (%) and the ratio of Treg cells (%), respectively. The cells were determined as a positive cell or negative cell based on the maximum fluorescence intensity in the negative control. Namely, the cells showing higher fluorescence intensity than the maximum fluorescence intensity of the negative control were determined as a positive cell, while the cells showing a fluorescence intensity equal to or lower than the maximum fluorescence intensity of the negative control were determined as a negative cell. The ratio of positive cells was calculated as the ratio of the number of positive cells relative to the number of total cells.

**[Table 9]**

| Measurement sample | Polypeptide marker | Remark |
|---|---|---|
| 1. (2) | PTPRM positive and MCAM positive | New marker set |
| 1. (3) | PTPRM positive and L1CAM positive | New marker set |
| 1. (4) | PTPRM positive and CCR6 positive | New marker set |
| 1. (5) | PTPRM positive and CXCR3 negative | New marker set |
| 1. (6) | CCR6 positive and CXCR3 negative | Known marker set |
| 1. (7) | CCR6 positive and MCAM positive | New marker set |
| 1. (8) | CCR6 positive and L1CAM positive | New marker set |

### 4. Correlation analysis of each Th17 marker and Th positive cells

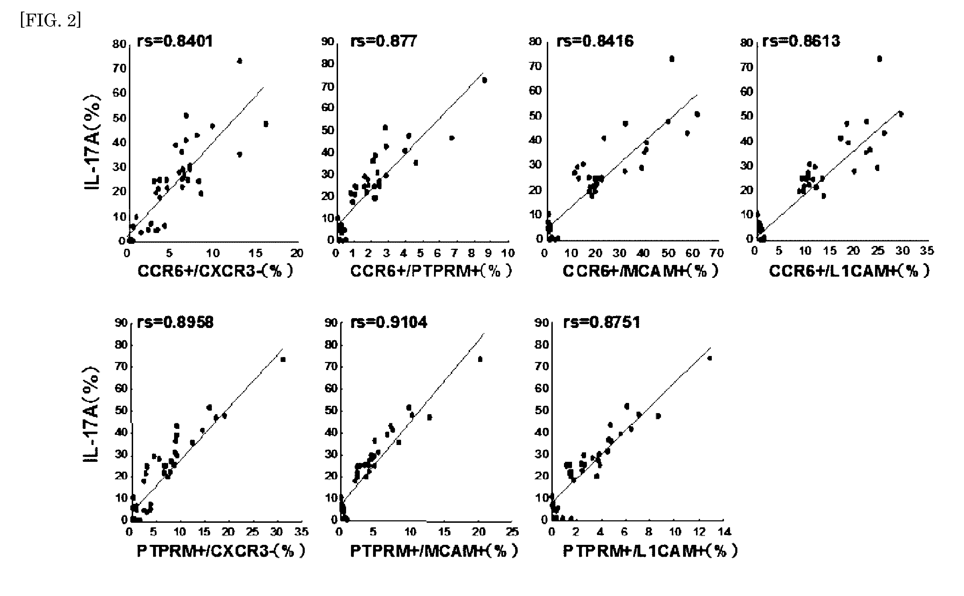

Spearman's rank-correlation coefficient of the ratio of the number of cells expressed by polypeptide markers obtained in the above 3 (%) and the ratio of the number of IL-17A positive cells (i.e., the ratio of Th17 cells) (%) was calculated by Staxflex software (Artech Co.,Ltd.). In the same manner, Spearman's rank-correlation coefficient of the ratio of the number of cells expressed by polypeptide markers (%) and the ratio of the number of IFN-γ positive cells (i.e., the ratio of Th1 cells) (%), the ratio of the number of IL-4 positive cells (i.e., the ratio of Th2 cells) (%) or the ratio of the number of FOXP3 positive cells (i.e., the ratio of Treg cells) (%) was calculated. Spearman's rank-correlation coefficients in the combinations of markers (marker sets) are shown in Table 10. In addition, the plot of "the ratio of the number of cells expressed by each polypeptide marker (%)" against "the ratio of Th17 cells detected by IL-17A (%)" for each sample is shown in Fig. 2. In Fig. 2, "rs" means Spearman's rank-correlation coefficient. Also, "+" and "-" put after the name of each marker means that "the marker is expressed (positive)" and "the marker is not expressed (negative)".

**[Table 10]**

| Market set | | Known marker | | | |
|---|---|---|---|---|---|
| | | L1-17A (Th17) | IFN-γ (Th1) | IL-4 (Th2) | FOXP3 (Treg) |
| Known | CCR6/CXCR3 | 0.8401 | -0.1113 | -0.2957 | 0.5608 |
| New | CCR6/PTPRM | 0.8770 | 0.0604 | -0.3919 | 0.4530 |
| | CCR6/MCAM | 0.8416 | 0.0556 | -0.7392 | 0.1281 |
| | CCR6/L1CAM | 0.8613 | 0.1570 | -0.7177 | 0.1745 |
| | PTPRM/CXCR3 | 0.8958 | -0.0848 | -0.3601 | 0.4559 |
| | PTPRM /MCAM | 0.9104 | 0.1751 | -0.5736 | 0.2992 |
| | PTPRM / L1CAM | 0.8751 | 0.1948 | -0.5370 | 0.2935 |

It is found from Fig. 2 and Table 10 that the combinations of the markers for detecting Th17 cells of the present invention have a high correlation with the detection method using IL-17A marker that is a standard detection method of Th17 cells, in the same level or more as CCR6 and CXCR3 that is a known marker set.
On the other hand, the combinations of the marker for detecting Th17 cells of the present invention have no correlation with the methods of detecting Th1, Th2 and Treg cells (methods using INF-γ, IL-4 and FOXP3 markers).
This suggests that the marker for detecting Th17 cells of the present invention can specifically detect Th17 cells. For example, by using the marker of the present invention, it is considered that Th17 cells can be specifically detected in a sample containing cells such as tissue collected from a patient or the like, furthermore, it is considered that the marker can be used for the determination of whether or not the patient is affected with a disease considered to be involved in Th17 cells, for example, autoimmune disease such as RA.

### (Example 4)

Analysis of the ratio of Th17 cells contained in fractions of each marker for detecting Th17 cells

### (1) Preparation of fraction samples

From the mixed samples of Th cells 2 (sample with a mixing ratio of Th1:Th2:Treg:Th17 = 0.5:1:1:1) prepared in 1. (1) of Example 3, samples for measuring PTPRM/MCAM, PTPRM/CCR6, CCR6/CXCR3 and CCR6/L1CAM polypeptide markers were respectively prepared by the methods similar to 1. (2), (4), (6) and (8) of Example 3.
From Th cells in the prepared samples for measuring each polypeptide marker, PTPRM positive and MCAM positive cells, PTPRM positive and CCR6 positive cells, CCR6 positive and CXCR3 positive cells and CCR6 positive and L1CAM positive cells were each separated using a cell sorter (FACS Aria: Becton Dickinson and Company).
These separated cells were each seeded in a 96 well plate in a density of 1.5 x 10⁵ cells or less/0.3 ml/well. The cells were cultured in Yssel medium (IMDM, 1% human serum of AB-type, 0.25% BSA, 1.8 mg/l 2-aminomethanol, 40 mg/l transferrin, 5 mg/l insulin, 2 mg/l linoleic acid, 2 mg/l oleic acid, 2 mg/l palmitic acid, 1% penicillin/ streptomycin) to which IL-2 (R&D systems, Inc.) was added so as to have a final concentration of 10 ng/ml in an incubator at 37°C in an atmosphere of 5% CO₂ for three days, to prepare PTPRM/MCAM fraction sample, PTPRM/CCR6 fraction sample, CCR6/CXCR3 fraction sample and CCR6/L1CAM fraction sample.

### (2) Sample treatment for IL-17A positive cell measurement

In order to measure IL-17A positive cells contained in each of the mixed sample of Th cells 2 before fraction in the above (1), PTPRM/MCAM fraction sample, PTPRM/CCR6 fraction sample, CCR6/CXCR3 fraction sample and CCR6/L1CAM fraction sample after fraction, IL-17A measurement samples were prepared from each sample. Specifically, the mixed sample of Th cells 2, PTPRM/MCAM fraction sample, PTPRM/CCR6 fraction sample, CCR6/CXCR3 fraction sample and CCR6/L1CAM fraction sample were prepared so as to be 5% FBS/RPMI at 2.5 x 10⁵ cells/ml. Subsequently, phorbol myristate acetate and ionomycin were added so as to have a final concentration of 50 ng/ml and a final concentration of 1 µM, respectively, and incubated at 37°C for 4 hours to stimulate Th cells. Then, brefeldin A was added so as to have a final concentration of 10 µg/ml and incubated at 37°C for 2 hours.
After cultivation, phosphate buffered saline (PBS) containing 0.5% BSA was added, and the mixture was centrifuged to collect Th cells, thereby washing the cells. The washed Th cells were added with 2% paraformaldehyde to fix the cells. After fixing the cells, a saponin buffer (0.5% saponin, 0.5% bovine serum albumin (BSA), 1 mM sodium azide (in PBS)) was added to accelerate cell membrane permeability of Th cells.
To the sample after saponin buffer treatment was added a PerCP-Cy5.5-labeled anti-IL-17A antibody (eBioscience, Inc.), and the mixture was reacted at 4°C for 20 minutes.
After the reaction, the saponin buffer was added, and the mixture was centrifuged to collect Th cells, thereby washing the cells. The washed Th cells were suspended in PBS containing 0.5% BSA to prepare an IL-17A measurement sample.

### (3) Measurement of IL-17A positive cells

The IL-17A measurement samples prepared in the above (2) were analyzed using FACSCanto II (BD biosciences) and FACS DIVA software (BD biosciences). In IL-17A measurement samples, the ratio of the number of IL-17A positive cells to the number of total cells, the ratio of Th17 cells (%), was calculated.
The cells were determined as a positive cell or negative cell based on the maximum fluorescence intensity in the negative control. Namely, the cells showing higher fluorescence intensity than the maximum fluorescence intensity in the negative control were determined as a positive cell.
On the contrary, while the cells showing a fluorescence intensity equal to or lower than the maximum fluorescence intensity in the negative control were determined as a negative cell. The ratio of positive cells was calculated as the ratio of the number of positive cells relative to the number of total cells.

### (4) Calculation of Th17 concentration rate

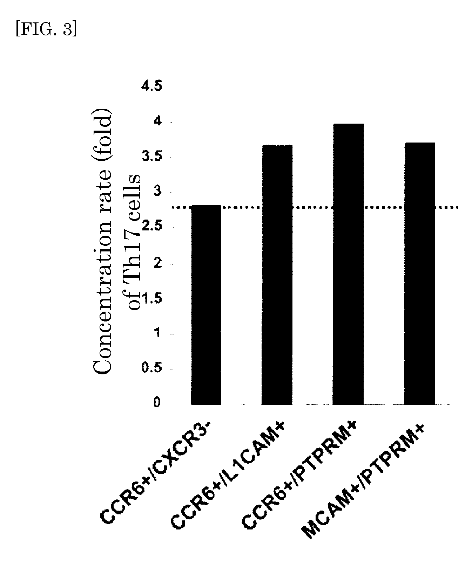

The Th17 concentration rate in the fractions of marker for detecting Th17 cells was calculated, based on the ratio of Th17 cells (%) of samples obtained in the above (3). Specifically, the ratios of the ratio of Th17 cells (%) of PTPRM/MCAM fraction sample, PTPRM/CCR6 fraction sample, CCR6/CXCR3 fraction sample and CCR6/L1CAM fraction sample, to the ratio of Th17 cells (%) of the mixed samples of Th cells 2 obtained in the above (3) were respectively calculated. The obtained ratios were defined as Th17 concentration rate, and the Th17 concentration rates in the fractions of marker for detecting Th17 cells were shown in Fig. 3.
It can be seen from Fig. 3 that, in the combinations of the markers for detecting Th17 cells of the present invention of "CCR6 and PTPRM", "CCR6 and L1CAM" and "MCAM and PTPRM", Th17 cells are concentrated in a concentration higher than CCR6 and CXCR3 that is a conventional marker set. Based on the above, it can be seen that Th17 cells can be isolated from a sample containing various human-derived cells with higher accuracy than ever before by using the marker of the present invention.

## Claims

1. A marker for detecting human IL-17-producing helper T cells, comprising a polynucleotide having a nucleotide sequence of a gene represented by at least one selected from the group consisting of L1CAM, MCAM and PTPRM or a variant or a fragment thereof.

2. The marker for detecting human IL-17-producing helper T cells according to claim 1, further comprising a polynucleotide having a nucleotide sequence of a gene represented by at least one selected from the group consisting of CCR6 and CXCR3 or a variant or a fragment thereof.

3. A marker for detecting human IL-17-producing helper T cells, comprising a protein encoded by a gene represented by at least one selected from the group consisting of L1CAM, MCAM and PTPRM or a functionally equivalent variant or a fragment thereof.

4. The marker for detecting human IL-17-producing helper T cells according to claim 3, comprising a protein encoded by a gene represented by at least one selected from the group consisting of CCR6 and CXCR3 or a functionally equivalent variant or a fragment thereof.

5. A method for detecting human IL-17-producing helper T cells comprising steps of
obtaining an expression level of the marker for detecting human IL-17-producing helper T cells according to any one of claims 1 to 4, in a sample containing cells, and
detecting human IL-17-producing helper T cells in the sample, based on the obtained expression level of the marker.

6. The detection method according to claim 5, wherein the step of obtaining the expression level of the marker is steps of
bringing a labeled antibody specifically reacting with the marker for detecting human IL-17-producing helper T cells that is labeled with a labeling substance into contact with the sample containing cells to prepare a measurement sample, and
detecting labeling of the labeled antibody bound to human IL-17-producing helper T cells through the marker for detecting human IL-17-producing helper T cells in the obtained measurement sample.

7. The detection method according to claim 6, wherein
the step of obtaining the expression level of the marker is a step of obtaining the expression levels of a plurality of the markers for detecting human IL-17-producing helper T cells different from each other, and
the labeled antibody is an antibody different from each other that specifically reacts with each of a plurality of the markers for detecting human IL-17-producing helper T cells, and is labeled with mutually distinguishable labeling substances.

8. The detection method according to claim 6 or 7, wherein the labeled antibody is an antibody labeled with a fluorescent labeling substance.

9. The detection method according to claim 8, wherein the fluorescence emitted from the fluorescently-labeled antibody is detected using a flow cytometer.
